# EUROPEAN PATENT SPECIFICATION

(11) **EP 3 082 576 B1**
(45) Date of publication and mention of the grant of the patent: **11.03.2020**
(21) Application number: 14825439.4
(22) Date of filing: 02.12.2014
(51) Int. Cl.: A61B 5/00, A61B 17/54

(54) **METHOD FOR TREATING THE SKIN AND DEVICE**
VERFAHREN ZUR BEHANDLUNG DER HAUT UND VORRICHTUNG
MÉTHODE DE TRAITEMENT DE LA PEAU ET DISPOSITIF

(30) Priority: 20.12.2013 US 201314137411
(43) Date of publication of application: 26.10.2016
(73) Proprietor: L'OREAL, 75008 Paris (FR)
(72) Inventor: GREZ, Joseph, North Bend, Washington 98045 (US)
(74) Representative: Nony
(86) International application number: PCT/IB2014/066505
(87) International publication number: WO 2015/092587

(56) References cited:
- WO-A2-01/41651
- US-A1- 2006 189 964
- US-A1- 2009 088 823
- US-A1- 2009 222 023
- US-A1- 2010 049 177
- US-A1- 2013 158 547
- US-B1- 6 629 983

## Description

### FIELD OF THE INVENTION

This invention pertains to skin treatment and is related to a vacuum assisted treatment of the skin.

### BACKGROUND OF THE INVENTION

At the micro level, skin has a highly textured folded morphology that creates difficulties in obtaining even coverage and exposure to treatments such as for cosmetic, mechanical action, etc.

Treatments often become concentrated in either the folds or treatments can be limited to the high-points leaving the folds untreated. However it is often important to address both areas uniformly.

Furthermore, properties of skin such as skin firmness highly depends on first the skin type and secondly the part of the body or the face to treat.

U.S. patent application No. 11/863 440 discloses an apparatus for treating the skin including a chamber positioned adjacent to the skin. A source of vacuum removes the air from the chamber. The treatment is performed by the application of an electromagnetic radiation to the skin through a transparent window on which the skin is drawn up.

U.S. patent application No. 61/081 110 discloses an applicator for RF, ultrasound or light treatment of the skin that comprises a hollow cavity formed inside a housing. The cavity communicates with a source of negative pressure. The pressure in the cavity sucks up the skin that comes into contact with a valve. The skin opens or closes the valve depending on the pressure in the cavity. The housing can comprise one of more RF electrodes and/or ultrasound transducers to treat the skin.

U.S. patent application No. 10/393 682 discloses a device for skin microdermabrasion configured to contact the skin. By application of vacuum, the skin is sucked up into a chamber to contact an abrasive member. Then the device is moved to abrade the skin in contact with the abrasive member.

US 6,629,983, WO 01/41651, US 2009/222023 and US 2010/049177 disclose methods and devices for skin abrasion using vacuum sources which apply constant vacuums to the skin.

There remains a need for improving methods and devices for evaluating the skin and/or for treating the skin.

### SUMMARY OF THE INVENTION

The invention is defined in independent claims 1 and 13 and their respective dependent claims.

For sucking up into the chamber a targeted area of the skin, the pressure into the chamber is decreased to be under the ambient atmospheric pressure to exert an axial force distributed over the exposed skin. The skin is deformed such that it protrudes in the chamber.

A "targeted area of the skin" is to be understood as the area of the skin that is in the chamber for treatment and/or for evaluation by the surface.

When sucked up into the chamber, the skin is stretched. The skin of the targeted area is at least partially flattened, on the sub-mm scale, to allow a more uniform application of treatments even on the folds.

Furthermore, the surface is protected from inadvertent contact with the skin or other external objects by virtue of its recessed position, which proves advantageous when the surface is made by sensitive structures such as micro-needles or an application pad. Contamination of the surface may be avoided this way, if desirable.

The mechanical action or the application of the composition by the surface is exerted while the skin is in contact with the surface.

Puncturing the skin creates pathways through the stratum corneum , the skin's main barrier to infusion, to improve the infusion of active ingredients in the skin. Therefore, puncturing the skin prepares the latter to the application of a composition containing at least one active ingredient. The flattening of the skin enables the puncturing to be performed uniformly without the need of deep puncturing which could hurt and generate bleeding. Shorter micro-needles may be used.

Abrading the skin enables the removal of dead cells from the outermost layer of the skin to provide a healthier looking appearance, wrinkle reduction, to clean out blocked pores, or the treatment of undesirable skin conditions to enhance skin tone. Abrading is performed with accuracy so that the skin tissue is not damaged while the dead cells are removed effectively.

The abrading may be performed by a movement of the surface. The amplitude and speed of the friction of the skin on the surface may be selected in response to the skin morphology and firmness.

Alternatively, the abrading may be performed by the repeated movement of the skin. Indeed, while the skin is oscillating axially, the skin surface area in the chamber is alternatively increasing and decreasing and each small part of the skin within the chamber is alternatively and laterally stressed and relaxed. The dead cells may be removed this way. Furthermore, the skin may oscillate between an upper and a lower position, the skin of the targeted area contacting the surface at least in the upper position. As the skin comes into contact with the surface at least in the upper position, the skin of the targeted area is rubbed against the surface, improving the abrading.

The invention may enable exfoliating the folds in skin structure.

Measuring an extent of the contact area and/or of the contact pressure 1) enables active adaption of the pressure in the chamber depending on the desired treatment, 2) enables control of the treatment progress depending on results of measurement, and 3) may provide information representative of the firmness of the skin as loose skin tends to be sucked up higher than firm skin.

This enables measurement of elasticity of the skin.

Preferably the targeted area of the skin is a region of the face or the body. The invention may allow preferential treatment of thinner or more wrinkled skin when compared to thicker less wrinkled skin. For example, loose skin such as the corners of the eyes and forehead where wrinkles have started, for example for subjects older than 50 year old, are pulled deeper into the chamber than tight area of the skin. This allows selective treatment of looser areas of the skin automatically, depending on the depth of the surface, which may be selected, so that one skin type is accessed and another is not. This enables the selective application of cosmetics or physical treatments that could serve to tighten the skin in the right areas without treating areas not in need.

The method is not a therapeutic method and is a cosmetic method. When a composition is applied, the composition is preferably a cosmetic composition. The composition may be an anti-wrinkle composition, a moisturizer, a tightening composition.

The surface may or may not generate ultrasound waves or electric current, nor electromagnetic radiation such as light or heat.

Preferably, the relative pressure within the chamber is ranging from 2 to 10 mmHg under ambient atmospheric pressure during sucking up of the skin, better from 4 to 6 mmHg.

The skin of the targeted area can be treated without displacement of the chamber relative to the skin outside the chamber, especially when the skin is to be punctured. Between treatments of the targeted area, the chamber may be displaced, for example translated to another area of the skin, either manually or automatically.

The skin of the targeted area may be stretched during sucking up to a stretched surface extent which is less than twice the surface extent of the targeted area in an unstretched state prior to sucking up. Preferably, the skin is stretched from flat to a relatively smooth dome. The extent of surface is approximated by the ratio of the dome surface to the flat disk surface prior to sucking up. The ratio preferably ranges from 1.2 to 1.8.

The stretching of the skin preferably occurs at a sub-mm scale, not at a micron scale, only the natural creases in the surface of the skin being unfolded, not the specific structures of the skin at the micron size scale.

The depth of penetration of the skin in the chamber may range from 1 mm to 3 mm, preferably 1.5 mm to 2.5 mm.

While coming into contact with the surface, the skin of the targeted area may deform to flatten itself on the surface, all the surface of the targeted area tending to come into contact with the surface and to adapt its shape relative to the surface shape.

Preferably, the targeted area occupies when contacting the surface from 2 to 10 mm². The opening of the chamber may have a radius of about 4 mm, preferably ranging from 3 mm to 10 mm. The depth of penetration of the skin within the chamber may range from D/10 to D/2, better from D/8 to D/4, where D is a largest dimension of the opening through which skin is sucked up.

The targeted area may comprise at least one fold, this fold being flattened while the skin of the targeted area is sucked up and stretched. The fold is at least partially unfolded, allowing the treatment to be performed therein.

The method may comprise increasing the pressure within the chamber after treatment of the targeted area to cause the skin of the targeted area to move away from the surface. The increase of pressure can result from an opening of a valve or an action of a pressure source. The pressure may increase up to atmospheric pressure or over atmospheric pressure.

As mentioned above, the pressure cyclically decreases and increases to cause the targeted area of the skin to oscillate between an upper position and a lower position within the chamber. The oscillation of the skin causes an alternation of stress step and relax step of the skin which may increase the flexibility of the skin. The cycle frequency of the pressure is preferably ranging from 30 Hz to 80 Hz. The number of oscillations per second ranges for example from 5 to 100.

The pressure within the chamber may be increased to more than the ambient atmospheric pressure after treatment of the targeted area to ease displacement of the chamber relative to the skin towards another targeted area. The chamber may be displaced relative to the skin outside the chamber while the pressure in the chamber is greater than ambient atmospheric pressure. Therefore, the device floats on a cushion of air on the skin and can be moved easily without friction on the skin from a targeted area of the skin to another targeted area of the skin.

The surface, before sucking up of the skin of the targeted area within the chamber, may be at a distance from the targeted area ranging from 0.5 mm to 8 mm, better from 1 mm to 3 mm.

Preferably, the puncturing is performed by at least one micro-needle protruding from a remainder of the surface, better a plurality of micro-needles. The at least one micro-needle has preferably a height equal to or less than 30 µm, better equal to or less than 20 µm. Such a height enable the micro-needles to perforate the stratum corneum of the skin without penetrating deep into the tissue to avoid bleeding, pain and risk of infection. The puncturing may extend substantially only through the stratum corneum, not into the dermis. The puncturing may be followed by application of a cosmetic composition containing an active component intended to be absorbed.

The abrading may result from a lateral and/or rotational movement of the surface relative to the chamber, the movement possibly being oscillating. Thus, the surface may gently abrade the skin of the targeted area, removing the dead cells. The abrading may take place within the folds as well as on the high spots between folds. Interstitial debris may be knocked off. After treatment, the skin rebounds to its normal pillowed morphology again.

In a variant, the abrading results from an axial movement of the surface relative to the chamber, the axial movement possibly being oscillating up and down. Therefore the abrading is improved as the stretching of the skin of the targeted area is performed in contact with the surface. The surface may be displaced axially by the skin being sucked up.

The surface may be mounted on a spring and the targeted area of the skin may exert a force on the surface that deforms the spring, maintaining contact of skin on the surfaces as the skin stretches.

The application of the composition may be performed by a porous element of the surface impregnated with the composition. The porous element is preferably feeded with the composition taken from a reservoir of composition, the feeding possibly being continuous. The composition may be drawn from the reservoir thanks to the vacuum existing within the chamber. The stretching of the skin enables an even coverage of coating in folds as well as on the high spots between folds.

The recessed position of the surface may be spaced enough from an opening of the chamber through which the skin of the targeted area is sucked up to selectively treat only loose skin when multiple targeted areas are treated. As explained before, the behaviour of loose and firm skin may differ by the height each may possibly reach within the chamber at a same pressure within the chamber; loose skin protrudes higher than firm skin.

The method may comprise adjusting the distance from the opening of the chamber to the surface. For example, an adjustment member may be rotated to vary the distance, for example by extending a wall of the chamber defining an edge resting on the skin during treatment or turning a knob to which an internal element defining the surface is attached.

The extent of contact between the surface and the skin of the targeted area may be less than the extent of the area defined by the external perimeter of the targeted area. The skin present into the chamber may have regions having different firmnesses, the loose regions coming into contact with the surface and the firm regions remaining apart from the surface.

A same or a further composition may be sprayed on the targeted area within the chamber. The composition sprayed may uniformly coat the surface of the skin.

Ultrasound waves and/or electric current may be applied to the skin of the targeted area, possibly through or from the surface. The application of ultrasound waves may facilitate the removal of dead cells from the surface of the skin and generate sonophoresis to increase absorption of a composition into the skin. The application of an electric current may generate electrophoresis to increase absorption of a composition into the skin.

Electromagnetic radiation such as light or heat may be applied to the skin of the targeted area, possibly through or from the surface.

The surface may be heated above the ambient temperature. An extent of a contact area between the skin and the surface and/or a contact pressure between the skin and the surface may be measured. This may be useful for measuring elasticity of the skin, or to indicate to a user when a treatment for thin wrinkled skin has been successful, or for conditioning a treatment to the detection of a specific type of skin.

The surface may comprise a treating region for treatment of the skin and a probe region for measuring the extent of contact and/or the contact pressure. The treating region and the probe region may overlap at least partially, or may not overlap, being separate regions. For example the treating region and the probe region may both be centered on the surface and the probe region may extend around the treating region.

The treatment may occur only when the targeted area contacts with a predetermined extend the surface or when the skin's force against the surface exceeds a predetermined value. For example, ultrasonic waves or a pulse of light are only applied in case contact is detected. The surface may be rotated only when contact with the skin is detected, for a given duration, which may avoid over-abrading the skin.

The method may comprise determining whether a pressure exerted by the skin of the targeted area on the surface or an extent of contact area between the skin of the targeted area and the surface exceeds a threshold value. Treatment of the skin of the targeted area may be authorized when the pressure exerted by the skin of the targeted area on the surface or the extent of contact area between the skin of the targeted area and the surface exceeds the threshold value. The method may comprise exposing the skin of the targeted area to ultrasound waves using a transducer at least partially defining the surface, only if the pressure exerted by the skin of the targeted area or the extent of contact area between the skin of the targeted area and the surface exceeds the threshold value, to avoid damaging the transducer if not in proper contact with the skin.

The method may comprise detecting a quick fall in the contact pressure to determine when the micro-needles of the surface pierce the skin and thus when the penetration is completed.

Further examples disclosed relate to a method for evaluating skin firmness or a surface morphology of the skin, comprising:
- sucking up into a chamber positioned over the skin a targeted area of skin to bring the targeted area into contact with a probe surface present at a recessed position within the chamber, and
- determining based upon an extent of a contact area between the surface and the targeted area of the skin and/or a contact pressure between the surface and the targeted area of the skin a degree of skin firmness and/or an information useful for assessing skin morphology.

This evaluation may be performed before and/or after the treatment of the skin according to the method defined above to efficiently treat the skin without damaging it or hurting it. The evaluation may prove useful to determine a level of vacuum to apply and/or to set the position of the surface within the chamber.

The evaluation may also prove advantageous to demonstrate the role of a cosmetic treatment on skin firmness or morphology.

For example, skin elasticity is first assessed by measuring depth of penetration of the skin within the chamber, by detecting for example if there is or not contact with the surface and/or the extent of contact.

Then the skin is treated, for example by application of a cosmetic composition containing an anti-wrinkle active. Then skin elasticity is assessed again, and the results are compared to demonstrate a benefit of the composition for improving skin elasticity.

Further embodiments of the present invention relate to a device for treating and/or evaluating a skin of a targeted area, comprising:
- a chamber with an opening configured to face the targeted area of the skin when the device is applied on the skin,
- a surface within the chamber at a recessed position from the opening, and
- a pressure source in communication with the chamber, at least for decreasing the pressure in the chamber and for causing the targeted area to be sucked up in the chamber and to contact the surface,
the surface being configured for puncturing the skin of the targeted area and/or applying a composition to the skin of the targeted area by transfer from the surface,
and/or the surface comprising at least one probe to measure
- an extent of a contact area between the surface and the targeted area of skin,
   and/or
- a contact pressure between the surface and the targeted area of skin.

Such a device may be used to perform the methods defined above.

Further embodiments of the present invention relate to a device for treating a skin of a targeted area, comprising:
- a chamber with an opening configured to face the targeted area of the skin when the device is applied on the skin,
- a surface within the chamber at a recessed position from the opening, and
- a pressure source in communication with the chamber, at least for decreasing the pressure in the chamber and causing the targeted area to be sucked up in the chamber and to contact the surface,
the surface being configured for abrading the skin of the targeted area, the surface being mobile relative to the chamber, and/or
the device comprising a control member for controlling the pressure source and to cause a repeated movement of the skin of the targeted area within the chamber between an upper and a lower position, the skin of the targeted area contacting the surface at least in the upper position.

The pressure source may also create an overpressure within the chamber to ease repositioning of the chamber relative to the skin toward another targeted area.

The opening may be of circular shape. The diameter of the opening may range from 6 mm to 10 mm. Such diameter may be given by the inner diameter of the chamber.

Preferably, the pressure source is configured to decrease the pressure within the chamber down to 2 to 10 mmHg under ambient atmospheric pressure during sucking up of the skin, better from 4 to 6 mmHg. The pressure source may be controllable by adjusting the rotation speed of a pump rotor or a frequency of reciprocation of a reciprocating pump or a duty cycle of a valve communicating with a vacuum source.

The distance of the surface from the opening is preferably ranging from 0.5 mm to 8 mm, better from 1 mm to 3 mm. Such a distance may be adjustable, either manually or automatically, using for example a manual screw adjuster, a servomotor or a step motor.

The distance may depend from a largest dimension of the opening. The distance may range from D/10 to D/5, where D is a largest dimension of the opening, as mentioned above.

The distance may be adjusted based on the detection of the force exerted by the skin on the surface.

Accordingly, if the force of contact is low, the distance may be reduced so as to increase the area of contact.

The section of the opening may range from 6 mm to 10 mm as stated above.

The pressure source may be configured to increase the pressure within the chamber to more than the pressure of the ambient. The pressure source may be configured to cause the pressure to oscillate while the targeted area of the skin remains within the chamber. The frequency of oscillation may range from 5 Hz to 100 Hz, preferably from 30 Hz to 80 Hz.

Alternatively, the pressure may be increased by the controlled opening of a valve. The pressure within the chamber may oscillate while the targeted area of the skin remains within the chamber thanks to periodic opening and closing of the valve.

The device may comprise at least one micro-needle protruding from the surface, better a plurality of micro-needles protruding on the surface to puncture the skin of the targeted area. The micro-needles can have a height equal to or less than 30 µm, better equal to or less than 20 µm.

The surface may be laterally and/or rotationally mobile relative to the chamber, the movement possibly being oscillating and being driven by a motor, especially when the surface is abrading.

Alternatively, the surface is axially mobile relative to the chamber, the axial movement possibly being oscillating. The surface is preferably pushed by the skin being sucked up, preferably using a spring to return the surface in an initial position when the skin withdraws.

The movement of the surface may have an axial amplitude ranging from 0 to 3 mm.

The surface may comprise a treating region for treatment of the skin of the targeted area and a probe region for measuring the extent of contact and/or the contact pressure. The treating region and the probe region may overlap at least partially.

The surface may comprise a porous element impregnated with the composition. The device may comprise a reservoir containing a composition to deliver to the porous element, the reservoir being connected to the porous element by a delivery element. The flow of coating material may be generated by the decreasing in pressure within the chamber that sucked up the skin of the targeted area. This is advantageous because no flow will occur unless skin contact is made and it is therefore self regulating.

The surface may be flat, concave or convex towards the opening. Preferably, the surface is shaped to maximize the treatment of the skin of the targeted area.

The surface may be defined by an internal element that is interchangeable, depending on the treatment to perform.

The probe may comprise at least one of a force transducer to measure the pressure of skin on the surface, a capacitive or resistive sensor to measure a contact with the surface.

The device may comprise a spraying element to apply the same or a further composition on the targeted area.

The device may comprise at least one ultrasound transducer, at least one electrode and/or an electromagnetic source. The ultrasound transducer and/or the electrode may be configured to come into contact with the skin of the targeted area. The electromagnetic source may be a light source or a heating source and may be configured to treat the skin of the targeted area.

The surface may comprise a heating element to heat the surface, the heating element preferably being a resistor.

Further embodiments of the present invention relate to a method for spraying a composition on the human skin, comprising:
sucking a targeted area of skin up into a chamber,
spraying a composition to the skin of the targeted area

The spray of the composition to the skin of the targeted area is preferably generated while the targeted area is sucked up.

The invention may be better understood from reading the following detailed description of non-limiting implementation examples thereof, and from examining the appended drawing, in which:
- figure 1 schematically and partially shows a skin surface in an unstretched state,
- figure 2 is a cross-section of figure 1 showing the skin of figure 1 according to II-II,
- figure 3 is a schematic and partial cross-section of an example of a device according to the invention,
- figure 4 schematically shows an example of device according to the invention,
- figure 5 is a representation according to figure 3, the skin being in an upper position and contacting the surface,
- figure 6 is a representation according to figure 3, the skin being in a lower position,
- figure 7 schematically and partially shows an alternative of a device according to the invention,
- figure 8 shows the alternative of figure 7, the skin of the targeted area coming into contact with the surface,
- figure 9 shows the alternative of figure 8, the surface being moved by the skin of the targeted area,
- figures 10 and 11 are diagrams illustrating alternatives of pressure evolution in the chamber as a function of time,
- figure 12 is a diagram illustrating the contact of skin on the surface as a function of time,
- figure 13 is a schematic view of a probe surface from below,
- figure 14 is a schematic view of a variant of probe surface from below,
- figure 15 schematically and partially shows the device during displacement thereof,
- figure 16 is a schematically and partial view of a surface configured for puncturing,
- figures 17 to 22 are schematic and partial views of different variants of a device according the invention, and
- figure 23 shows a variant wherein a composition is sprayed onto the targeted area.

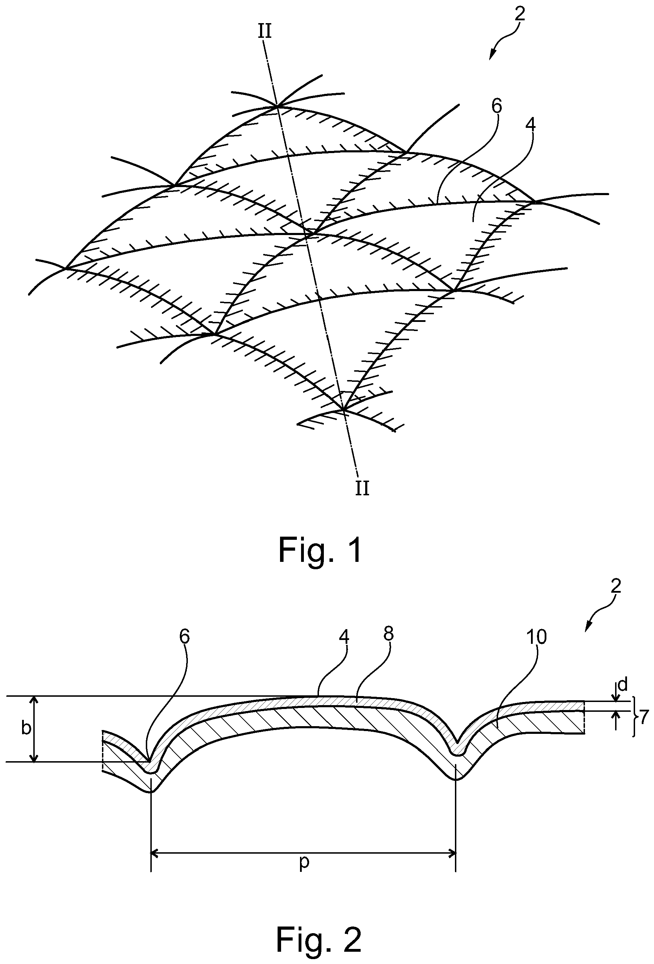

As illustrated on figure 1, skin surface 2 has a highly textured folded morphology at the micro level and in an unstretched state. The skin surface 2 comprises high areas 4 separated by folds 6.

As illustrated on figure 2, the depth *b* of folds 6 of skin surface 2 is usually ranging from 40 to 200 µm and high areas 4 have a maximum distance p between two folds ranging from 0.5 to 1 mm.

As shown on figure 2, the skin surface 2 is composed of the epidermis 7, the stratum corneum 8 disposed in contact with the exterior of the body and the viable epidermis 10 disposed under the stratum corneum 8. The thickness d of the stratum corneum 7 is usually ranging from 15 to 20 µm.

In the prior art, treatments, such as coating, mechanical stimulation or infusion, often become concentrated in either the folds 6 or are limited to the high areas 4 leaving the folds 6 untreated.

Examplary embodiments of the invention provide a device for physically flattening or un-folding the skin surface during treatment exposures, so that a more uniform application or treatment may be accomplished.

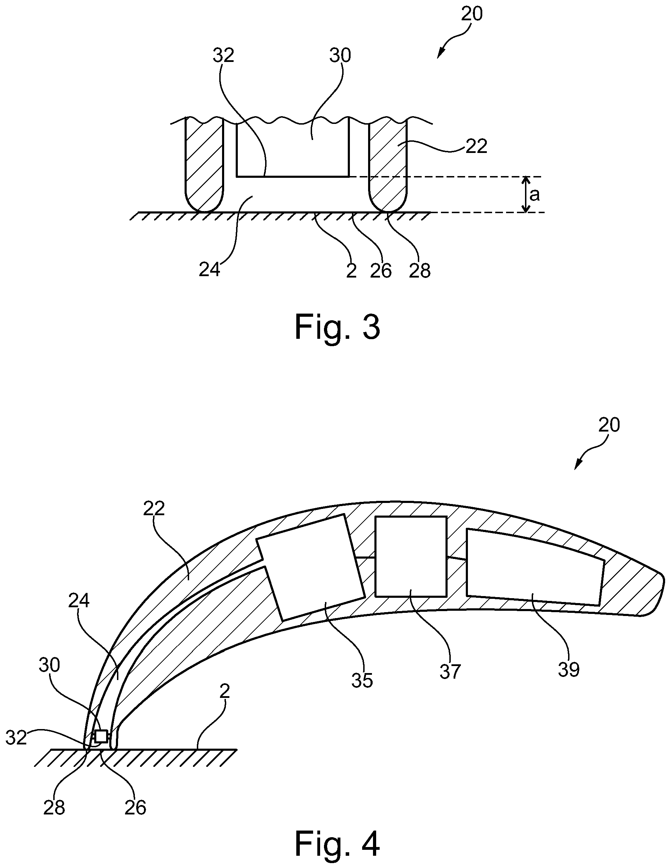

Such a device 20 is illustrated on figures 3 and 4 and described below.

The device 20 comprises a housing 22 defining a chamber 24 with an opening 26 intended to face the skin surface 2. The opening 26 is bounded by a rim 28 of the housing 22 configured to contact the skin surface 2, preferably in a substantially sealed manner, when the device is applied on the skin. The rim 28 may have an annular shape, of inside diameter of about 8 mm for example.

The device 20 further comprises an internal element 30 defining a surface 32 configured to treat and/or evaluate the skin surface 2. The internal element 30 is arranged within the chamber 24 such that the surface 32 is at a recessed position from the opening 26.

Preferably, the surface 32 is at a distance *a* from the opening 26 ranging from 0.5 mm to 8 mm, better from 1 mm to 3 mm, for example about 2 mm for a 8 mm opening.

The chamber 24 is in communication with a pressure source 35, shown on figure 4.

The internal element 30 allows the passage of air from the opening 26 to the pressure source 35.

The pressure source 35 may be controlled by a control circuit 37, being electrically linked to a power source 39.

As illustrated on figure 4, the device 20 may be a hand held device configured to be easily displaced on the skin surface 2. In a variant not shown, the device comprises a hand piece defining the opening of the chamber and a base station comprising a vacuum source.

The pressure source 35 is able to decrease the pressure into the chamber 22 at a value P that is less than the ambient atmospheric pressure Pₐₜₘ.

The decrease of pressure within the chamber sucks the skin up into the chamber. The low pressure tends to increase contact pressure between the rim 28 and the skin surface 2 and the resulting friction helps prevent the skin from outside the opening 26 to slide into the chamber 24.

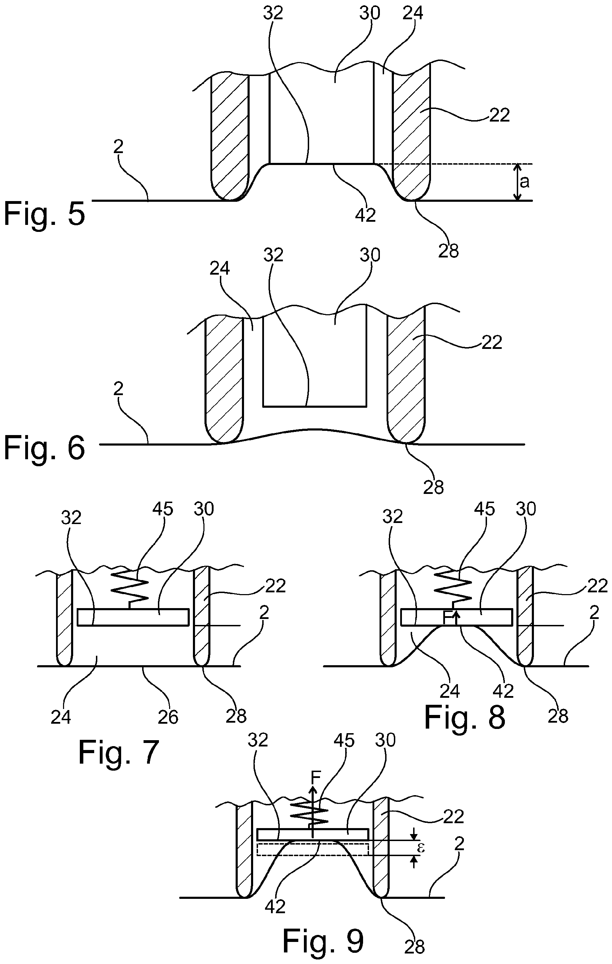

As illustrated on figure 6, the skin facing the opening 26 takes a dome shape within the chamber 24. The skin facing the opening 26 is stretched and the folds 6 are flattened at the sub-mm scale.

In extreme situations, the surface of the skin facing the opening may be stretched such that the skin fills totally the internal space of the chamber 24. The skin is preferably stretched up to less than three times its initial area in the un-stretched state, better less than twice, more preferably about 1.5 times.

As illustrated on figure 5, while progressively decreasing the pressure within the chamber, the skin progressively deforms and comes into contact with the surface 32, exposing the folds 6 and the high areas 4 substantially evenly to the surface 32.

The rim 28 has preferably rounded edges to facilitate the sucking up of the skin through the opening 26 and displacement of the housing on the skin between treatments.

The surface 32 has preferably a circular contour but another shape is possible.

The surface 32 may be flat or may be concave or convex towards the skin. It may have a cup shape that substantially matches the dome shape of the skin.

The absolute maximum pressure difference relative to the ambient atmospheric pressure given by ΔPₘₐₓ₌Pₐₜₘ-Pₘᵢₙ is ranging preferably from 2 to 10 mmHg, better from 4 to 6 mmHg when the skin of the targeted area 42 is contacting the surface 32.

The internal element 30 may be fixed within the chamber 24 or be mobile. The movement of the internal element 30 may be a lateral, a rotational or an axial movement, or a combination of these movements. When the movement is a rotational movement, the axis of rotation may be coaxial with the opening or not coaxial with the opening, for example perpendicular to the axis of the opening.

As illustrated on figures 7 to 9, the internal member 30 may be mounted on a spring 45 to be able to move axially up and down, accompanying the movement of the skin. Alternatively, movement may be off axis or rotary to provide a frictional motion.

As illustrated in figure 8, the skin of the targeted area 42 may come into contact with the surface 32 and exert a force F on the internal element 30. This force may be sensed using an appropriate sensor. This force may also cause the surface to move axially, provided the internal element is able to move when pushed by the skin.

As illustrated on figure 9, when the force F is sufficient, the internal element 30 may move up accompanied by the skin of the targeted area 42. The dashed lines represent the position of the internal element 30 before it moves. The internal element 30 may move up of a distance *ε* ranging from 0 and 3 mm.

The initial position of the surface may be adjusted based on the maximum force applied by the skin on the surface.

The initial position of the surface may also be adjusted so that the force does not exceed a predetermined value.

The device 20 may comprise a control system to control the pressure within the chamber 24.

The control system may be part of the control circuit 37 controlling the pump 35.

Alternatively, the control system may comprise a valve present on the housing 22 that can open to increase the pressure within the chamber 24. Thus, the pressure within the chamber may be increased by opening the valve to release partially or totally the skin.

As illustrated on figure 6, the pressure P can be controlled to remain below ambient atmospheric pressure Pₐₜₘ while being above Pₘᵢₙ so that the skin remains deformed within the chamber while not contacting the surface 32. This may occur when the skin is oscillated between high and low positions and contacts the surface 32 in the high position, as described below.

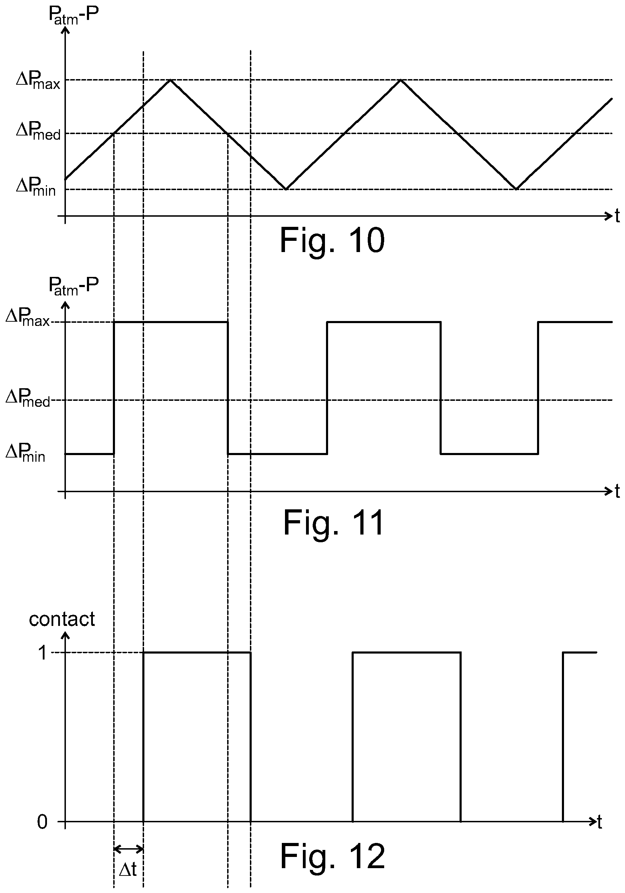

As illustrated on figures 10 and 11, the pressure within the chamber 24 can be controlled to cyclically decrease and increase such that the targeted area 42 of the skin oscillates between an upper position and a lower position.

The lower position may correspond to the skin in an unstretched state.

To oscillate the skin alternatively to a stressed and relaxed state may increase its flexibility. Furthermore, the oscillation of the skin favors the removing of the dead cells on the skin and may increase the clarity and luster of the skin.

The relative pressure to the ambient atmospheric pressure ΔP in the chamber may oscillate between an upper pressure ΔPₘₐₓ and a lower pressure ΔPₘₘ according to a triangle wave, as illustrated on figure 10 or a square wave, as illustrated on figure 11, or a complex wave. The invention is not limited to a particular waveform.

As illustrated on figure 12, the skin can come into contact (at 1) with the surface 32 in the upper position and come apart (at 0) from the surface 32 in the lower position. The skin may respond to the pressure oscillation with a delay *Δt* as illustrated on figure 12.

The surface 32 may comprise at least one probe to evaluate the skin morphology and firmness. The surface 32 may only comprise a probe 48 and not be intended for treating the skin or may both carry a probe and be intended for treating the skin.

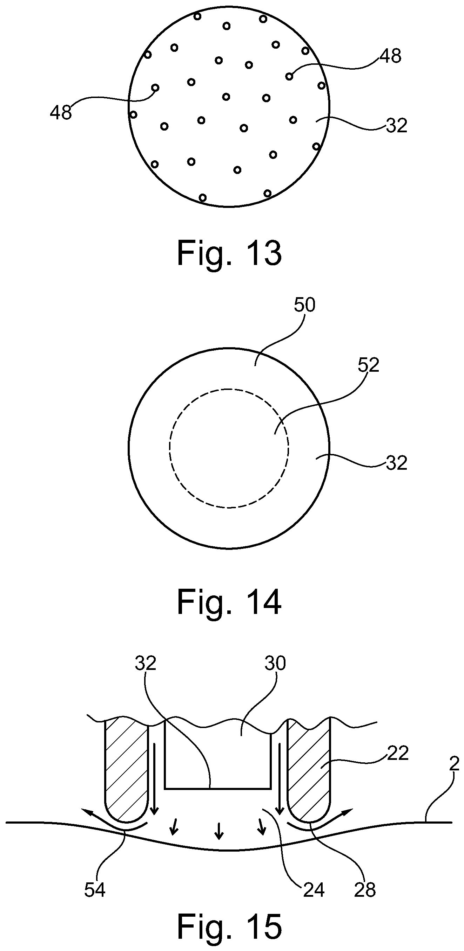

As illustrated on figure 13, the surface 32 may comprise a probe comprising a matrix of sensors 48 that detects the presence and extent of contact with the skin surface 2.

Each sensor may be an electrical sensor that detects when the skin is at its contact. In a variant, the sensors may be optical sensors that for example detect a change of refraction index when the skin comes into contact with the sensor.

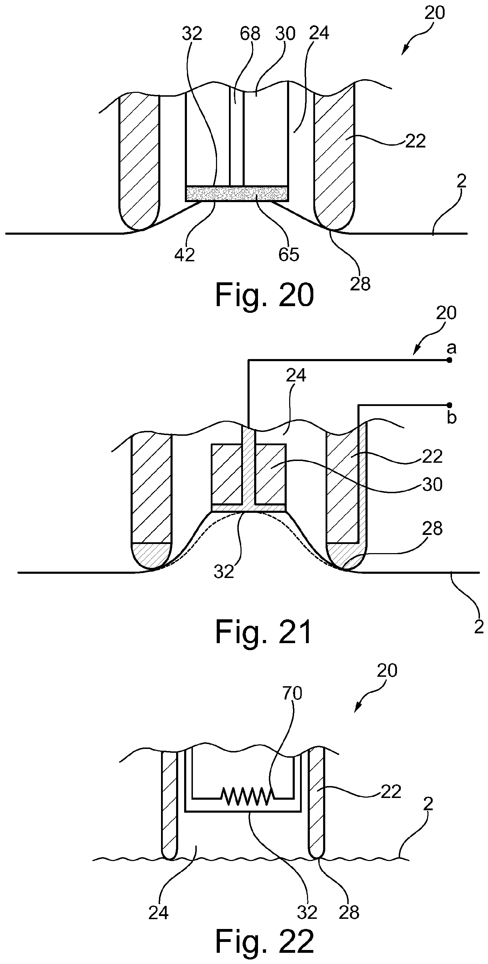

The surface 32 may comprise at least one probe 48 to measure the pressure of the skin against the surface 32. The probe 48 may comprise a force transducer. The pressure of the skin on the surface may also be measured through measurement of the displacement of the internal element 30 within the chamber 24, as detailed above. The probe 48 may be a capacitive or a resistive sensor, such as a resistor element, that has an output that is relational to the amount of area of skin in contact with the surface 32 as illustrated on figure 21. This may enable to assess skin firmness.

As illustrated on figure 21, the electrical capacity and impedance can be measured between two points *a* and *b, a* being linked to the surface 32 and *b* being linked to the rim 28 of the housing 22. The dotted line corresponds to the skin being sucked up to come into contact with the surface 32 in one point and the dashed line corresponds to the skin being sucked up to be in a full contact with the surface 32, both generating a signal on the sensor.

The surface 32 may be heated above ambient temperature. As illustrated on figure 22, the internal heating element 30 may be buried below the surface or the surface 32 may carry a heating element 70, preferably a resistor.

The surface 32 may comprise a probe 48 as described is U.S. patent No. 6 944 491 or as described in U.S. patent application No. 2004/0171962 hereby incorporated by reference in their entirety. The probe may be a non-optical probe comprising a plurality of individual detection cells forming a matrix to deliver an image of the surface. The image may be processed to provide information about a number and size of folds of the skin in contact with the surface 32.

As illustrated on figure 13, the surface 32 may comprise sensors 48 extending over the entire surface 32.

In the variant shown in figure 14, the probe region 50 extends only in a peripheral region of the surface 32.

The probe may help to determine when to apply a treatment and/or to stop a treatment. For example, only when the skin of the targeted area 42 is detecting as being in contact with the surface 32, the targeted area may be treated.

The surface 32 comprises at least one treatment region 52 to, for example, abrade, puncture, or apply a composition, in addition to the probe region 50.

Preferably, as illustrated on figure 14, the treatment region 52 extends at least centrally.

When the treatment has been applied, the pressure within the chamber may be increased as described before to a pressure higher or equal to the ambient atmospheric pressure.

Thus the skin is released, and the device 20 may be moved on the skin to treat another targeted area 42.

Preferably, as illustrated on figure 15, the pressure within the chamber 24 is increased to more than the ambient atmospheric pressure such that the skin may be moved away easily, the device 20 floating on a cushion of air 54 coming from the chamber 24 without friction on the skin.

The treatment carried by the surface 32 may be a puncturing treatment to improve later infusion of a product within the skin, preferably a cosmetic product, especially to help the product to go through the stratum corneum 8.

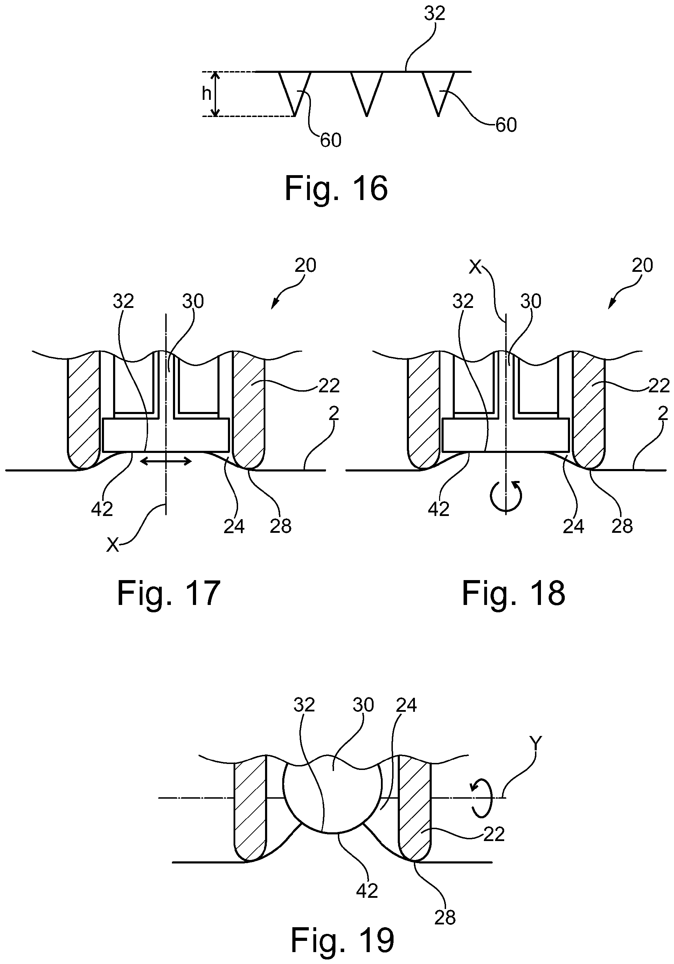

As illustrated on figure 16, the surface 32 may comprise at least one micro-needle 60, better a plurality of micro-needles 60.

Preferably, the micro-needles 60 are configured to penetrate only on the stratum corneum 8 to avoid bleeding and pain. Preferably, the micro-needles have a height *h* equal to or less than 30 µm, better equal to or less than 20 µm.

The micro-needles 60 require that the skin applies a force above a threshold force to penetrate the skin surface 2. Such a force can be measured to detect a fall down of the pressure exerted by the skin of the targeted area 42 against the surface 32, which corresponds to the moment where the micro-needles 60 pierce the skin surface 2. Once penetration is detected, pressure can be increased, to allow the skin to rebound to its initial flat state.

Preferably, the device 20 is immobilized during abrading relative to the skin outside the chamber 24 to avoid damaging the skin of the targeted area or the micro-needles 60 during treatment.

The treatment can also be an abrading treatment to remove dead cells from the skin surface 2. The abrasive surface 32 may be formed for example by fusing abrasive particles to a substrate, or could alternatively be made as an abrasive disk fixed on the support. Other removable and/or replaceable configurations are possible as well.

At least part of the internal element 30 may be mobile relative to the housing 22 to abrade the skin of the targeted area 42.

As illustrated on figures 17 and 18, the movement of the internal element 30 may be traverse to the axis of the chamber or rotational along an axis X. The transverse movement may be reciprocating movement.

Alternatively, as illustrated on figure 19, the internal element 30 has a round shape and may rotate according to at least one axis Y, which may be perpendicular to the axis of the opening.

Alternatively, the skin may oscillate within the chamber as described above while contacting the surface 32. Therefore, the skin undergoes small lateral movement while the skin is stretched in contact with the surface 32 such that the skin is laterally rubbed onto the surface 32 and thus abraded.

The treatment may comprise applying a composition on the skin, preferably a cosmetic composition.

As illustrated on figure 20, the surface 32 may comprise a porous element 65 attached to a support.

The porous element 65 may be impregnated with the composition and/or may be fed by composition from a reservoir through a delivery element, such as a duct 68.

All skins and all part of the body are not equal in firmness and morphology. There are different skin-types. For example wrinkled and non-wrinkled areas such as loose skin as fine line areas below the eye or forehead where wrinkles have started are moved deeper into the interior volume of the chamber 22 than firm skin as skin on the cheek.

The embodiments of the invention allow automatic selective treatment of specific areas by a consumer without attention to technique or process. This may be accomplished by imbedding the surface 32 at a critical depth where one skin type is accessed and another is not. This enables the selective application of cosmetics or physical treatments that could serve to treat the skin in just the right areas, without treating areas not in need.

The device 20 may comprise a spray to apply the same or a further composition on the skin into the chamber 24.

The device 20 may also comprise at least an ultrasound transducer, at least one electrode or an electromagnetic source such as a light source or a heating source, to apply respectively ultrasound waves, electric current and/or electromagnetic radiation.

Alternatively, the surface may constitute one electrode having a potential different from a potential of the targeted area of the skin before contact between the targeted area and the surface.

The surface may be connected to one terminal of an electrical sensor or generator, an other terminal of the electrical sensor or generator being connected to the skin, preferably through a rim defining at least partially the chamber. The generator may apply DC or AC current.

The surface 32 may comprise an ultrasound transducer that comes into contact with the skin when the latter is sucked up and at least a probe that measure the extent of the skin of the targeted area 42 on the surface 32 at least around the transducer. Therefore, the ultrasound waves are generated only when the probe detects the pressure of the skin around the transducer, to avoid emitting ultrasound energy in the absence of contact with the skin, which may destroy the transducer.

The measure of the extent of contact between the surface 32 and the skin of the targeted area 42 may give information about the firmness of the skin. Indeed, loose skins are sucked up higher than firm skins.

The probe may allow to determine the morphology of the skin surface 2 to evaluate if the skin is stretched enough to flatten all folds and treat efficiently the skin. Such a measurement may be made dynamically or not, after sucking up. The measurement may be made dynamically to evaluate how the skin reacts to the stress and determine skin-type.

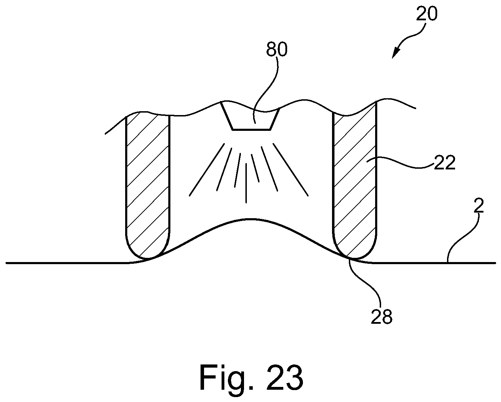

Alternatively, as illustrated on figure 23, the device 20 comprises a spraying element 80 instead of an internal element 30. The spraying element 80 is configured to spray a composition on the skin of the targeted area when stretched. The skin of the targeted area does not contact the spraying element 80. This allows the application of a composition on the whole surface of the skin, even in the folds.

The invention is not limited to the embodiments described above and illustrated on the drawings. The shape of the chamber may vary, as well as the shape of the surface for treating the skin.

The expression "comprising a" should be understood as being synonymous to "comprising at least one".

## Claims

1. A cosmetic method for treating the human skin, comprising:
sucking a targeted area of skin, in particular a region of the face or the body, up into a chamber to bring the targeted area into contact with a surface present at a recessed position within the chamber so as to exert at least one of several possible mechanical actions to the targeted area, including abrading the skin at the targeted area, the abrading resulting from repeated movement of the skin against the surface in response to repeating vacuum cycles, the repeated movement of the skin being between an upper and a lower position, the skin of the targeted area contacting the surface at least in the upper position.

2. Method according to claim 1, the skin being sucked up into the chamber to bring the targeted area into contact with the surface present at a recessed position within the chamber so as to,
- abrade the skin by a relative movement of the surface relative to the chamber while in contact with the targeted area, and/or
- apply a composition to the targeted area by transfer from the surface, and/or
- exert puncturing of the skin of the targeted area, and/or
- measure an extent of a contact area between the surface and the targeted area of the skin, and/or
- measure a contact pressure between the surface and the targeted area of the skin.

3. The method of claim 1 or 2, wherein the relative pressure within the chamber is ranging from 2 to 10 mmHg under ambient atmospheric pressure during sucking up of the skin, better from 4 to 6 mmHg.

4. The method according to any of the preceding claims, wherein skin of the targeted area is:
- treated without displacement of the chamber relative to the skin outside the chamber, and/or
- stretched during sucking up to a stretched surface extent which is less than twice the surface extent of the targeted area in an unstretched state prior to sucking up, and/or
- flattened against the treating surface, and/or
wherein the targeted area comprises at least one fold, the fold being stretched while the skin of the targeted area is sucked up.

5. The method according to any of the preceding claims, comprising
- increasing a pressure within the chamber after treatment of the targeted area to cause the skin of the targeted area to move away from the surface, the pressure within the chamber being increased to more than the ambient pressure after treatment of the targeted area to ease lateral displacement of the chamber relative to the skin towards another targeted area, and/or
- cyclically decreasing and increasing a pressure within the chamber to cause the targeted area of the skin to oscillate between an upper position and a lower position within the chamber, the cycle frequency of the pressure preferably ranging from 1 Hz to 100 Hz.

6. The method according to any of the preceding claims, wherein the surface is, before sucking up the skin of the targeted area, at a distance from the targeted area ranging from 0.5 mm to 8 mm, better from 1 mm to 3 mm.

7. The method according to any of the preceding claims, the skin being sucked up into the chamber to bring the targeted area into contact with the surface present at a recessed position within the chamber so as to apply a composition to the targeted area by transfer from the surface, wherein the surface comprises a porous element impregnated with the composition, the porous element being preferably fed with the composition taken from a reservoir of composition, the feeding possibly being continuous, and the composition being preferably transferred by vacuum in said chamber.

8. The method according to any of the preceding claims, wherein the recessed position of the surface is spaced from an opening of the chamber through which the skin of the targeted area is sucked up so as to selectively treat only loose skin when multiple targeted areas are treated.

9. The method according to any of the preceding claims, comprising
- applying to the skin of the targeted area, possibly through the surface, ultrasound waves, and/or an electrical current, and/or electromagnetic radiation, especially light and/or heat.

10. The method according to any of the preceding claims, the skin being sucked up into the chamber to bring the targeted area into contact with the surface present at a recessed position within the chamber so as to,
- abrade the skin by a relative movement of the surface relative to the chamber while in contact with the targeted area, and/or
- apply a composition to the targeted area by transfer from the surface, and/or
- exert puncturing of the skin of the targeted area, and/or
- measure an extent of a contact area between the surface and the targeted area of the skin, and/or
- measure a contact pressure between the surface and the targeted area of the skin.

11. The method according to any of the preceding claims, wherein the surface comprises a treating region for treatment of the skin and a probe region for measuring the extent of contact and/or the contact pressure, preferably the treating region and the probe region overlapping at least partially.

12. The method according to any of the preceding claims, wherein the surface constitutes one electrode having a potential different from a potential of the targeted area of the skin before contact between the targeted area and the surface, and/or wherein the surface is connected to one terminal of an electrical probe, an other terminal of the electrical probe being connected to the skin, preferably through a rim defining at least partially the chamber.

13. Device for treating skin of a targeted area, comprising:
- a chamber with an opening, preferably a circular opening, configured to face the targeted area of the skin when the device is applied on the skin,
- a surface, preferably that is flat, concave or convex towards the opening, within the chamber at a recessed position from the opening, a distance of the surface from the opening being preferably ranging from 0.5 mm to 8 mm, better from 1 mm to 3 mm, and
- a pressure source in communication with the chamber, at least for decreasing the pressure in the chamber and causing the targeted area to be sucked up in the chamber and to contact the surface, the pressure source being preferably configured to decrease the pressure within the chamber down to 2 to 10 mmHg under ambient atmospheric pressure during sucking up of the skin, better to 4 to 6 mmHg, and/or to increase the pressure in the chamber to more than the pressure of the ambient atmospheric pressure, and/or to cause the pressure to oscillate while the targeted area of the skin remains within the chamber, the surface being configured for abrading the skin of the targeted area, the device comprising a control member for controlling the pressure source to cyclically decrease and increase the pressure within the chamber to as to cause repeated movement of the skin of the targeted area within the chamber between an upper and a lower position, the skin of the targeted area contacting the surface at least in the upper position,

14. The device according to claim 13, the surface being configured to:
- be mobile relative to the chamber, and/or
- apply a composition to the skin of the targeted area by transfer from the surface, and/or
- puncture the skin of the targeted area and/or
- measure, with a probe it contains,
• an extent of a contact area between the surface and the targeted area of skin, and/or
• a contact pressure between the surface and the targeted area of skin, preferably the at least one probe being at least one of a force transducer to measure the pressure of skin on the surface, a capacitive sensor to measure the axial displacement of the surface and/or an electrical sensor to measure the extent of the skin of the targeted area contacting the surface.

15. The device according to claim 13 or 14, comprising
- a treating region for treatment of the skin of the targeted area and a probe region for measuring the extent of contact and/or the contact pressure, preferably the treating region and the probe region overlapping at least partially, and/or
- a porous element impregnated with the composition, preferably, the device comprising a reservoir containing the composition to deliver to the porous element, the reservoir being connected to the porous element by a delivery element, and/or
- a spraying element to apply the same or a further composition on the targeted area, and/or
- at least one ultrasound transducer, and/or electrodes configured to come into contact with the skin of the targeted area, and/or a heating element to heat the surface, the heating element preferably being a resistor.

16. The device according any one of claims 13 to 15, wherein the surface is laterally and/or rotationally mobile relative to the chamber, the movement possibly being oscillating and being driven by a motor, and/or the surface is axially mobile relative to the chamber, the axial movement possibly being oscillating, the surface being preferably pushed by the skin being sucked up, preferably using a spring.

## Patentansprüche

1. Kosmetisches Verfahren zur Behandlung der menschlichen Haut, umfassend:
Ansaugen eines Zielbereichs der Haut, insbesondere eines Bereichs des Gesichts oder des Körpers, in eine Kammer, um den Zielbereich in Kontakt mit einer Oberfläche zu bringen, die sich in einer vertieften Position innerhalb der Kammer befindet, um mindestens eine von mehreren möglichen mechanischen Aktionen auf den Zielbereich auszuüben, einschließlich Abschleifen der Haut auf dem Zielbereich, wobei das Abschleifen aus wiederholter Bewegung der Haut gegen die Oberfläche als Reaktion auf wiederholte Vakuumzyklen folgt, der wiederholten Bewegung der Haut zwischen einer oberen und einer unteren Position, wobei die Haut des Zielbereichs die Oberfläche mindestens in der oberen Position kontaktiert.

2. Verfahren nach Anspruch 1, wobei die Haut in die Kammer angesaugt wird, um den Zielbereich in Kontakt mit der Oberfläche zu bringen, die sich in einer vertieften Position innerhalb der Kammer befindet, um
- die Haut durch eine Relativbewegung der Oberfläche relativ zur Kammer abzuschleifen, während sie mit dem Zielbereich in Kontakt ist, und/oder
- eine Zusammensetzung auf den Zielbereich durch Übertragung von der Oberfläche aufzutragen, und/oder
- Punktieren der Haut des Zielbereichs auszuüben und/oder
- ein Ausmaß einer Kontaktfläche zwischen der Oberfläche und der Zielfläche der Haut zu messen und/oder
- einen Kontaktdruck zwischen der Oberfläche und dem Zielbereich der Haut zu messen.

3. Verfahren nach Anspruch 1 oder 2, wobei der relative Druck innerhalb der Kammer im Bereich von 2 bis 10 mm Hg unter Umgebungsluftdruck während des Ansaugens der Haut, besser von 4 bis 6 mm Hg liegt.

4. Verfahren nach einem der vorhergehenden Ansprüche, wobei die Haut des Zielbereichs:
- ohne Verschiebung der Kammer gegenüber der Haut außerhalb der Kammer behandelt wird, und/oder
- während des Ansaugens auf einem gestreckten Oberflächenausmaß gestreckt wird, der weniger als das Doppelte des Oberflächenausmaßes des Zielbereichs in einem nicht gestreckten Zustand vor dem Ansaugen ist, und/oder
- gegen die Behandlungsoberfläche abgeflacht wird, und/oder
wobei der Zielbereich mindestens eine Falte umfasst, wobei die Falte gestreckt wird, während die Haut des Zielbereichs angesaugt wird.

5. Verfahren nach einem der vorhergehenden Ansprüche, umfassend:
- Erhöhen eines Drucks innerhalb der Kammer nach Behandlung des Zielbereichs, um zu bewirken, dass sich die Haut des Zielbereichs von der Oberfläche wegbewegt, wobei der Druck innerhalb der Kammer auf mehr als den Umgebungsdruck nach Behandlung des Zielbereichs erhöht wird, um seitliche Verschiebung der Kammer relativ zur Haut zu einem anderen Zielbereich zu erleichtern, und/oder
- zyklisches Verringern und Erhöhen eines Drucks innerhalb der Kammer, um zu bewirken, dass der Zielbereich der Haut zwischen einer oberen Position und einer unteren Position innerhalb der Kammer oszilliert, wobei die Zyklusfrequenz des Drucks vorzugsweise im Bereich von 1 Hz bis 100 Hz liegt.

6. Verfahren nach einem der vorhergehenden Ansprüche, wobei die Oberfläche, vor dem Ansaugen der Haut des Zielbereichs, in einem Abstand von dem Zielbereich liegt, der im Bereich von 0,5 mm bis 8 mm, besser von 1 mm bis 3 mm liegt.

7. Verfahren nach einem der vorhergehenden Ansprüche, wobei die Haut in die Kammer angesaugt wird, um den Zielbereich in Kontakt mit der Oberfläche zu bringen, die sich in einer vertieften Position innerhalb der Kammer befindet, um eine Zusammensetzung auf den Zielbereich durch Übertragung von der Oberfläche aufzubringen, wobei die Oberfläche ein poröses Element umfasst, das mit der Zusammensetzung imprägniert ist, wobei das poröse Element vorzugsweise mit der Zusammensetzung aus einem Reservoir der Zusammensetzung zugeführt wird, wobei die Zufuhr möglicherweise kontinuierlich ist, und die Zusammensetzung vorzugsweise durch Vakuum in die Kammer übertragen wird.

8. Verfahren nach einem der vorhergehenden Ansprüche, wobei die vertiefte Position der Oberfläche von einer Öffnung der Kammer beabstandet ist, durch die die Haut des Zielbereichs angesaugt wird, um selektiv nur lose Haut zu behandeln, wenn mehrere Zielbereiche behandelt werden.

9. Verfahren nach einem der vorhergehenden Ansprüche, umfassend:
- Aufbringen von Ultraschallwellen und/oder elektrischem Strom und/oder elektromagnetischer Strahlung, insbesondere Licht und/oder Wärme, auf die Haut des Zielbereichs, möglicherweise über die Oberfläche.

10. Verfahren nach einem der vorhergehenden Ansprüche, wobei die Haut in die Kammer angesaugt wird, um den Zielbereich in Kontakt mit der Oberfläche zu bringen, die sich in einer vertieften Position innerhalb der Kammer befindet, um
- die Haut durch eine Relativbewegung der Oberfläche relativ zur Kammer abzuschleifen, während sie mit dem Zielbereich in Kontakt ist, und/oder
- Zusammensetzung auf den Zielbereich durch Übertragung von der Oberfläche aufzutragen, und/oder
- Punktieren der Haut des Zielbereichs auszuüben und/oder
- ein Ausmaß einer Kontaktfläche zwischen der Oberfläche und der Zielfläche der Haut zu messen und/oder
- einen Kontaktdruck zwischen der Oberfläche und dem Zielbereich der Haut zu messen.

11. Verfahren nach einem der vorhergehenden Ansprüche, wobei die Oberfläche einen Behandlungsbereich zur Behandlung der Haut und einen Sondenbereich zur Messung des Ausmaßes des Kontakts und/oder des Kontaktdrucks umfasst, vorzugsweise wobei der Behandlungsbereich und der Sondenbereich sich mindestens teilweise überlappen.

12. Verfahren nach einem der vorhergehenden Ansprüche, wobei die Oberfläche eine Elektrode mit einem Potential darstellt, das sich von einem Potential des Zielbereichs der Haut vor dem Kontakt zwischen dem Zielbereich und der Oberfläche unterscheidet, und/oder wobei die Oberfläche mit einem Anschluss einer elektrischen Sonde verbunden ist, wobei ein anderer Anschluss der elektrischen Sonde mit der Haut verbunden ist, vorzugsweise durch einen Rand, der die Kammer mindestens teilweise definiert.

13. Vorrichtung zur Behandlung der Haut eines Zielbereichs, umfassend:
- eine Kammer mit einer Öffnung, vorzugsweise einer kreisförmigen Öffnung, die konfiguriert ist, um dem Zielbereich der Haut zugewandt zu sein, wenn die Vorrichtung auf die Haut aufgebracht wird,
- eine Oberfläche, die vorzugsweise zur Öffnung flach, konkav oder konvex ist, innerhalb der Kammer in einer von der Öffnung vertieften Position, wobei ein Abstand der Oberfläche von der Öffnung vorzugsweise im Bereich von 0,5 mm bis 8 mm, besser von 1 mm bis 3 mm liegt, und
- eine Druckquelle in Kommunikation mit der Kammer, mindestens um den Druck in der Kammer zu verringern und zu bewirken, dass der Zielbereich in der Kammer angesaugt wird und die Oberfläche zu kontaktieren, wobei die Druckquelle vorzugsweise konfiguriert ist, um den Druck innerhalb der Kammer nach unten auf 2 bis 10 mm Hg unter atmosphärischem Umgebungsdruck beim Ansaugen der Haut zu verringern, besser 4 bis 6 mm Hg, und/oder den Druck in der Kammer auf mehr als den Druck des atmosphärischen Umgebungsdrucks zu erhöhen und/oder zu bewirken, das der Druck oszilliert, während der Zielbereich der Haut innerhalb der Kammer bleibt,
wobei die Oberfläche konfiguriert ist, um die Haut des Zielbereichs abzuschleifen, wobei die Vorrichtung ein Steuerelement zum Steuern der Druckquelle umfasst, um den Druck innerhalb der Kammer zyklisch zu verringern und zu erhöhen, um wiederholte Bewegung der Haut des Zielbereichs innerhalb der Kammer zwischen einer oberen und einer unteren Position zu bewirken, wobei die Haut des Zielbereichs die Oberfläche mindestens in der oberen Position kontaktiert.

14. Vorrichtung nach Anspruch 13, wobei die Oberfläche konfiguriert ist, um:
- relativ zur Kammer beweglich zu sein und/oder
- eine Zusammensetzung auf die Haut des Zielbereichs durch Übertragung von der Oberfläche aufzutragen, und/oder
- die Haut des Zielbereichs zu punktieren und/oder
- mit einer Sonde, die sie enthält,
• ein Ausmaß einer Kontaktfläche zwischen der Oberfläche und der Zielfläche der Haut zu messen und/oder
• einen Kontaktdruck zwischen der Oberfläche und dem Zielbereich der Haut zu messen, vorzugsweise wobei die mindestens eine Sonde mindestens ein Kraftwandler ist, um den Druck der Haut auf die Oberfläche zu messen, ein kapazitiver Sensor, um die axiale Verschiebung der Oberfläche zu messen und/oder ein elektrischer Sensor, um das Ausmaß der Haut des Zielbereichs zu messen, der die Oberfläche kontaktiert.

15. Vorrichtung nach Anspruch 13 oder 14, umfassend
- einen Behandlungsbereich zur Behandlung der Haut des Zielbereichs und einen Sondenbereich zur Messung des Kontaktausmaßes und/oder des Kontaktdrucks, vorzugsweise wobei der Behandlungsbereich und der Sondenbereich sich mindestens teilweise überlappen, und/oder
- ein mit der Zusammensetzung imprägniertes poröses Element, vorzugsweise wobei die Vorrichtung ein Reservoir umfasst, das die Zusammensetzung zur Abgabe an das poröse Element enthält, wobei das Reservoir durch ein Abgabeelement mit dem porösen Element verbunden ist, und/oder
- ein Sprühelement zum Aufbringen derselben oder einer weiteren Zusammensetzung auf den Zielbereich und/oder
- mindestens einen Ultraschallwandler und/oder Elektroden, die konfiguriert sind, um mit der Haut des Zielbereichs in Kontakt zu kommen, und/oder ein Heizelement, um die Oberfläche zu erwärmen, wobei das Heizelement vorzugsweise ein Widerstand ist.

16. Vorrichtung nach einem der Ansprüche 13 bis 15, wobei die Oberfläche relativ zur Kammer seitlich und/oder drehbar beweglich ist, die Bewegung möglicherweise oszillierend ist und von einem Motor angetrieben wird, und/oder die Oberfläche relativ zur Kammer axial beweglich ist wobei die axiale Bewegung möglicherweise oszillierend ist, wobei die Oberfläche vorzugsweise durch Ansaugen der Haut, vorzugsweise unter Verwendung einer Feder, gedrückt wird.

## Revendications

1. Procédé cosmétique pour traiter la peau humaine, comprenant :
l'aspiration d'une zone ciblée de peau, en particulier d'une région du visage ou du corps, dans une chambre pour amener la zone ciblée en contact avec une surface présente au niveau d'une position en retrait à l'intérieur de la chambre de sorte à exercer au moins une de plusieurs actions mécaniques possibles sur la zone ciblée, y compris l'abrasion de la peau au niveau de la zone ciblée, l'abrasion résultant d'un mouvement répété de la peau contre la surface en réponse à des cycles de vide répétés, le mouvement répété de la peau étant entre une position supérieure et une position inférieure, la peau de la zone ciblée entrant en contact avec la surface au moins dans la position supérieure.

2. Procédé selon la revendication 1, la peau étant aspirée dans la chambre pour amener la zone ciblée en contact avec la surface présente au niveau d'une position en retrait à l'intérieur de la chambre de sorte à
- abraser la peau par un mouvement relatif de la surface par rapport à la chambre alors qu'elle est en contact avec la zone ciblée, et/ou
- appliquer une composition sur la zone ciblée par transfert depuis la surface, et/ou
- exercer une ponction de la peau de la zone ciblée, et/ou
- mesurer une étendue d'une zone de contact entre la surface et la zone ciblée de la peau, et/ou
- mesurer une pression de contact entre la surface et la zone ciblée de la peau.

3. Procédé selon la revendication 1 ou 2, dans lequel la pression relative à l'intérieur de la chambre va de 2 à 10 mmHg sous pression atmosphérique ambiante pendant l'aspiration de la peau, mieux de 4 à 6 mmHg.

4. Procédé selon l'une quelconque des revendications précédentes, dans lequel la peau de la zone ciblée est :
- traitée sans déplacement de la chambre par rapport à la peau à l'extérieur de la chambre, et/ou
- étirée pendant l'aspiration jusqu'à une étendue de surface étirée qui est inférieure à deux fois l'étendue de surface de la zone ciblée dans un état non étiré avant l'aspiration, et/ou
- aplatie contre la surface de traitement, et/ou dans lequel la zone ciblée comprend au moins un pli, le pli étant étiré alors que la peau de la zone ciblée est aspirée.

5. Procédé selon l'une quelconque des revendications précédentes, comprenant
- l'augmentation d'une pression à l'intérieur de la chambre après traitement de la zone ciblée pour amener la peau de la zone ciblée à s'écarter de la surface, la pression à l'intérieur de la chambre étant augmentée à une pression supérieure à la pression ambiante après traitement de la zone ciblée pour faciliter un déplacement latéral de la chambre par rapport à la peau vers une autre zone ciblée, et/ou
- la réduction et l'augmentation cyclique d'une pression à l'intérieur de la chambre pour amener la zone ciblée de la peau à osciller entre une position supérieure et une position inférieure à l'intérieur de la chambre, la fréquence de cycle de la pression allant de 1 Hz à 100 Hz.

6. Procédé selon l'une quelconque des revendications précédentes, dans lequel la surface est, avant aspiration de la peau de la zone ciblée, à une distance de la zone ciblée allant de 0,5 mm à 8 mm, mieux de 1 mm à 3 mm.

7. Procédé selon l'une quelconque des revendications précédentes, la peau étant aspirée dans la chambre pour amener la zone ciblée en contact avec la surface présente au niveau d'une position en retrait à l'intérieur de la chambre de sorte à appliquer une composition à la zone ciblée par transfert depuis la surface, dans lequel la surface comprend un élément poreux imprégné avec la composition, l'élément poreux étant de préférence fourni avec la composition prélevée d'un réservoir de composition, la fourniture étant probablement continue, et la composition étant de préférence transférée par vide dans ladite chambre.

8. Procédé selon l'une quelconque des revendications précédentes, dans lequel la position en retrait de la surface est écartée d'une ouverture de la chambre à travers laquelle la peau de la zone ciblée est aspirée de sorte à traiter sélectivement uniquement la peau relâchée lorsque de multiples zones ciblées sont traitées.

9. Procédé selon l'une quelconque des revendications précédentes, comprenant
- l'application à la peau de la zone ciblée, probablement à travers la surface, d'ondes ultrasonores, et/ou d'un courant électrique, et/ou d'un rayonnement électromagnétique, en particulier de lumière et/ou de chaleur.

10. Procédé selon l'une quelconque des revendications précédentes, la peau étant aspirée dans la chambre pour amener la zone ciblée en contact avec la surface présente au niveau d'une position en retrait à l'intérieur de la chambre de sorte à
- abraser la peau par un mouvement relatif de la surface par rapport à la chambre alors qu'elle est en contact avec la zone ciblée, et/ou
- appliquer une composition à la zone ciblée par transfert depuis la surface, et/ou
- exercer une ponction de la peau de la zone ciblée, et/ou
- mesurer une étendue d'une zone de contact entre la surface et la zone ciblée de la peau, et/ou
- mesurer une pression de contact entre la surface et la zone ciblée de la peau.

11. Procédé selon l'une quelconque des revendications précédentes, dans lequel la surface comprend une région de traitement pour traitement de la peau et une région de sonde pour mesurer l'étendue de contact et/ou la pression de contact, la région de traitement et la région de sonde se chevauchant de préférence au moins partiellement.

12. Procédé selon l'une quelconque des revendications précédentes, dans lequel la surface constitue une électrode ayant un potentiel différent d'un potentiel de la zone ciblée de la peau avant contact enter la zone ciblée et la surface, et/ou dans lequel la surface est connectée à une borne d'une sonde électrique, une autre borne de la sonde électrique étant connectée à la peau, de préférence à travers un rebord définissant au moins partiellement la chambre.

13. Dispositif de traitement de la peau d'une zone ciblée, comprenant :
- une chambre avec une ouverture, de préférence une ouverture circulaire, configurée pour faire face à la zone ciblée de la peau lorsque le dispositif est appliqué sur la peau,
- une surface, qui est de préférence plate, concave ou convexe vers l'ouverture, à l'intérieur de la chambre au niveau d'une partie en retrait à partir de l'ouverture, une distance de la surface à partir de l'ouverture allant de préférence de 0,5 mm à 8 mm, mieux de 1 mm à 3 mm, et
- une source de pression en communication avec la chambre, au moins pour réduire la pression dans la chambre et amener la zone ciblée à être aspirée dans la chambre et à entrer en contact avec la surface, la source de pression étant de préférence configurée pour réduire la pression à l'intérieur de la chambre jusqu'à 2 à 10 mmHg sous pression atmosphérique ambiante pendant l'aspiration de la peau, mieux jusqu'à 4 à 6 mmHg, et/ou pour augmenter la pression dans la chambre à une pression supérieure à la pression atmosphérique ambiante, et/ou pour amener la pression à osciller alors que la zone ciblée de la peau reste à l'intérieur de la chambre,
la surface étant configurée pour abraser la peau de la zone ciblée, le dispositif comprenant un élément de contrôle destiné à contrôler la source de pression pour réduire et augmenter cycliquement la pression à l'intérieur de la chambre de sorte à amener un mouvement répété de la peau de la zone ciblée à l'intérieur de la chambre entre une position supérieure et une position inférieure, la peau de la zone ciblée entrant en contact avec la surface au moins dans la position supérieure.

14. Dispositif selon la revendication 13, la surface étant configurée pour :
- être mobile par rapport à la chambre, et/ou
- appliquer une composition à la peau de la zone ciblée par transfert depuis la surface, et/ou
- ponctionner la peau de la zone ciblée et/ou
- mesurer, avec une sonde qu'il contient
• une étendue d'une zone de contact entre la surface et la zone ciblée de peau, et/ou
• une pression de contact entre la surface et la zone ciblée de peau,
de préférence l'au moins une sonde étant au moins l'un d'un transducteur de force pour mesurer la pression de la peau sur la surface, d'un capteur capacitif pour mesurer le déplacement axial de la surface et/ou d'un capteur électrique pour mesurer l'étendue de la peau de la zone ciblée en contact avec la surface.

15. Dispositif selon la revendication 13 ou 14, comprenant
- une région de traitement pour le traitement de la peau de la zone ciblée et une région de sonde pour mesurer l'étendue de contact et/ou la pression de contact, la région de traitement et la région de sonde se chevauchant de préférence au moins partiellement, et/ou
- un élément poreux imprégné avec la composition, le dispositif comprenant de préférence un réservoir contenant la composition à délivrer à l'élément poreux, le réservoir étant raccordé à l'élément poreux par un élément de distribution, et/ou
- un élément de pulvérisation pour appliquer la même ou une autre composition sur la zone ciblée, et/ou
- au moins un transducteur ultrasonore, et/ou des électrodes configurées pour venir en contact avec la peau de la zone ciblée, et/ou un élément chauffant pour chauffer la surface, l'élément chauffant étant de préférence une résistance.

16. Dispositif selon l'une quelconque des revendications 13 à 15, dans lequel la surface est mobile latéralement et/ou en rotation par rapport à la chambre, le mouvement étant éventuellement oscillant et étant entraîné par un moteur, et/ou la surface est mobile axialement par rapport à la chambre, le mouvement axial étant éventuellement oscillant, la surface étant de préférence poussée par la peau étant aspirée, de préférence au moyen d'un ressort.
